(19) **Europäisches Patentamt / European Patent Office / Office européen des brevets**

(11) **EP 2 931 239 B1**

(12) **EUROPEAN PATENT SPECIFICATION**

(45) Date of publication and mention
of the grant of the patent:
**06.11.2019 Bulletin 2019/45**

(51) Int Cl.:
**A61K 8/49** *(2006.01)*          **A61Q 1/06** *(2006.01)*
**A61Q 1/12** *(2006.01)*

(21) Application number: **13826870.1**

(22) Date of filing: **17.12.2013**

(86) International application number:
**PCT/IB2013/061017**

(87) International publication number:
**WO 2014/097134 (26.06.2014 Gazette 2014/26)**

(54) **COSMETIC COMPOSITION COMPRISING WHITE NATURAL PIGMENTS HAVING HIGH COVERAGE**

KOSMETISCHE ZUSAMMENSETZUNG MIT WEISSEN NATÜRLICHEN PIGMENTEN MIT HOHER DECKKRAFT

COMPOSITION COSMÉTIQUE COMPRENANT DES PIGMENTS BLANCS NATURELS À POUVOIR COUVRANT ÉLEVÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2012 FR 1262159**

(43) Date of publication of application:
**21.10.2015 Bulletin 2015/43**

(73) Proprietor: **L'Oréal**
**75008 Paris (FR)**

(72) Inventors:
• **DELATTRE, Nathalie**
**F-78120 Sonchamp (FR)**
• **THEVENET, Ludovic**
**91490 Dannemois (FR)**

(74) Representative: **Nony**
**11 rue Saint-Georges**
**75009 Paris (FR)**

(56) References cited:
**JP-A- 59- 067 285          US-A- 3 892 844**
**US-A1- 2005 257 718**

**Description**

The present invention relates to the field of cosmetic compositions for caring for and/or making up keratinous substances and is targeted in particular at providing a cosmetic composition having high coverage.

Cosmetic compositions, intended to be applied to keratinous substances and in particular to the skin, lips and/or nails, are commonly employed to modify the visual appearance properties of the area where they are applied.

Generally, they are used to tone down or stimulate a natural colouration of these keratinous substances, indeed even to confer specific colouring aspects on them.

To these ends, cosmetic compositions comprise coloured pigments, such as, for example, iron oxides. In order to provide the white colour, the most widely used pigment is titanium dioxide ($TiO_2$), a pigment generally of synthetic origin. Furthermore, this pigment proves to be particularly advantageous cosmetically, due to its very high covering power.

In point of fact, nowadays, consumers are increasingly looking for cosmetic products essentially composed of compounds which are "natural" or "of natural origin".

The term "natural compound" is understood to mean a compound which is obtained directly from the earth or the soil, or from plants or animals, via, if appropriate, one or more physical processes, such as, for example, a milling, a refining, a distillation, a purification or a filtration.

The term "compounds of natural origin" is understood to mean a natural compound which has been subjected to one or more additional chemical or industrial treatments, giving rise to modifications which do not affect the essential qualities of this compound, and/or a compound predominantly comprising natural constituents which have or have not been subjected to transformations as indicated above. Mention may be made, as non-limiting example of additional chemical or industrial treatment giving rise to modifications which do not affect the essential qualities of a natural compound, of those allowed by the controlling bodies, such as Ecocert (Reference system for biological and ecological cosmetic products, January 2003), or defined in recognized handbooks in the field, such as Cosmetics and Toiletries Magazine, 2005, Vol. 120, 9:10.

According to a specific embodiment of the invention, a compound is regarded as natural or of natural origin when it is predominantly composed of natural constituents, that is to say when the ratio by weight of the natural constituents to the non-natural constituents of which it is composed is greater than 1.

In terms of colourants, natural pigments, such as, for example, iron oxides, or certain natural dyes make it possible to meet these expectations. On the other hand, it remains necessary to identify a natural alternative to the synthetic $TiO_2$ pigments widely considered in the cosmetics field for providing a white colour.

Admittedly, the "historical" natural white pigment known to a person skilled in the art, which is calcium carbonate ($CaCO_3$), makes possible access to this colour. However, its use is not satisfactory in terms of covering power.

JP 59-067285 A discloses a process for the preparation of powder guanine by lyophilization.

US 3892844 A describes sunscreen compositions comprising micronized guanine.

US 2005/257718 A relates to a dispersion of a natural pearlescent pigment derived from fish that can comprise guanine crystals.

Consequently, there remains a need for a "natural" colourant or colourant "of natural origin" having a white colour and furthermore having a satisfactory covering power.

The present invention is targeted precisely at meeting this need.

Thus, the present text describes a cosmetic composition for caring for and/or making up keratinous substances comprising, in a physiologically acceptable medium, non-interferential particles of guanine having a size of less than 30 μm.

In particular, the present invention relates, according to a first aspect, to a cosmetic composition for caring for and/or making up keratinous substances comprising, in a physiologically acceptable medium, non-interferential particles of guanine of natural origin having a D50 volume-average size ranging from 1 to 15 μm, said non-interferential particles of guanine of natural origin being prepared according to a process consisting of the following stages:

- a stage of milling natural guanine particles in a powder mill by air jet mill or pin mill, and
- a stage of drying.

Within the meaning of the present invention, the expression "non-interferential particles" denotes any particle, the structure of which does not make possible the creation of a colour effect by interference of light rays which diffract and scatter differently according to the nature of the layers. Thus, these particles cannot exhibit colours which vary according to the angle of observation and the incidence of the light.

Unexpectedly, the inventors have thus found that the use of specific particles of guanine makes it possible precisely to obtain cosmetic compositions satisfying the abovementioned requirements.

According to the invention, the said guanine particles are of natural origin and originate in particular from fish scales.

Guanine crystals are one of the components of the cells present on fish scales. It is precisely the interferential multilayer structure of these crystals which confers, on these fish, the silvery, white and iridescent colour. This property of guanine crystals of returning light is already nowadays being taken advantage of in the cosmetics field to formulate cosmetic compositions having a shimmering effect.

Contrary to all expectations, the inventors have today found that a specific milling of these crystals of interferential guanine results in particles which are, on the one hand, devoid of interferential properties (described, in the remainder of the description, as "non-interferential") and, on the other land, particularly advantageous for their covering power. Furthermore, the particles of reduced sizes prove to be insensitive to the phenomenon of agglomeration conventionally observed with particles of the same size but deriving from other materials. The fact that the particles do not have a tendency to adhere to one another is particularly advantageous for their formulation in cosmetics. They disperse in individualized fashion and thus provide very satisfactory coverage.

The present text discloses to non-interferential particles of guanine having a size of less than 30 $\mu$m, preferably of less than 15 $\mu$m, with advantageously at least 95% of the said guanine particles exhibiting a Heywood sphericity parameter multiplied by the size of the particle of less than 65 $\mu$m, in particular of less than 60 $\mu$m and better still of less than 55 $\mu$m.

The present text describes a process for the preparation of guanine particles according to the invention, comprising in particular the following stages:

- a stage of milling the natural guanine particles in a powder mill, in particular an air jet mill; and
- a stage of drying, in particular by heating, preferably via placing in an oven at a temperature of greater than or equal to 60°C for a period of time ranging from 1 to 8 hours and/or by bringing into contact with a stream of dry air.

Advantageously, the guanine particles of the present invention are prepared without any chemical treatment.

In particular, the particles do not undergo any step of dissolution or precipitation.

According to one aspect, the present invention relates to a process for the preparation of non-interferential particles of guanine of natural origin having a D50 volume-average size ranging from 1 to 15 $\mu$m consisting of the following stages:

- a stage of milling the natural guanine particles in a powder mill by air jet mill or pin mill; and
- a stage of drying.

According to one aspect, the present invention relates to a cosmetic method for caring for and/or making up keratinous substances, characterized in that a cosmetic composition according to the invention is applied to the keratinous substances.

Within the meaning of the present invention, the term "keratinous substance" is understood to cover the skin, the mucous membranes, such as the lips, the nails and the keratinous fibres, such as the eyelashes and the hair. The skin, in particular the skin of the face, the lips and the nails are very particularly considered according to the invention.

The term "physiologically acceptable medium" is intended to denote a medium which is particularly suitable for the application of a composition of the invention to keratinous substances, in particular the skin and more particularly the skin of the face, the lips and the nails.

The physiologically acceptable medium is generally suited to the nature of the support on which the product has to be applied.

A composition according to the invention can be any type of cosmetic composition, such as a nail varnish, a foundation, a face powder, an eye shadow, a concealer, a blusher, a lipstick, a lip balm, a lip gloss, a lip pencil, an eye pencil, an eyeliner, a mascara, a body make-up product, a skin-colouring product, a care product, such as a care cream, or a tinted cream, preferably a nail varnish, foundation or lipstick.

A composition of the invention is in particular a composition intended to be applied to a keratinous substance, in particular the skin and more particularly the skin of the face, the lips and the nails.

## Guanine particles

As specified above, a composition as described in the present text comprises non-interferential particles of guanine having a size of less than 30 $\mu$m.

Guanine is one of the four nucleotides which are found in DNA and in RNA. It is a purine derivative of empirical formula $C_5H_5N_5O$, consisting of a fused ring of pyrimidine and of imidazole with conjugated double bonds.

Guanine exists in the natural form and is also accessible by chemical synthesis but at a significant cost. Consequently, the most commonly selected source consists of fish scales.

As already specified above, this natural guanine exists in the form of crystals having an interferential multilayer structure.

This multilayer structure, which consists of an alternation between layers of guanine of high refractive index (n = 1.8) and of water of low refractive index (n = 1.3), is the reason for the pearlescent appearance of guanine. On the other hand, it should be emphasized that, in its native form, the high water content of guanine weakens its covering power.

In the context of the present invention, the guanine under consideration advantageously derives from these natural crystals, but which have been subjected to a specific milling in order to form non-interferential particles having a size ranging from 1 $\mu$m to 15 $\mu$m.

Within the meaning of the invention, the term "size" of a particle is understood to mean its D50. The D50 or volume-average size corresponds to the particle size defined so that 50% by volume of the particles have a size of less than D50.

The volume-average size can be estimated by light scattering using a MasterSizer laser particle sizer from Malvern, the said particles to be evaluated being dispersed in a liquid medium, such as, for example, octyldodecyl neopentanoate.

Guanine particles may have a size ranging from 0.5 to 30 $\mu$m, more particularly of less than 20 $\mu$m and preferably of less than 15 $\mu$m.

According to the invention, the said guanine particles have a size ranging from 1 to 15 $\mu$m.

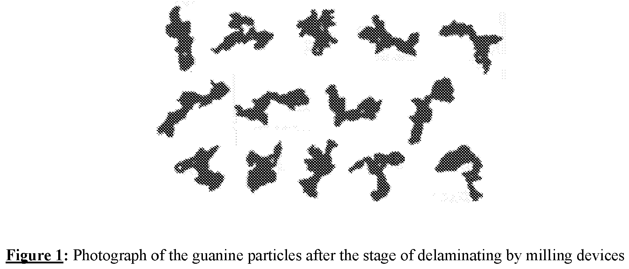

The guanine particles according to the present invention are not only smaller than the guanine crystals in their native form (D50 = 49 $\mu$m), are non-interferential and also advantageously have a highly characteristic shape represented in the appended Figure 1. The size of the particles is expressed in D50, it being assumed that they are spherical.

It is precisely in order to take into account the particular shape of the guanine particles in accordance with the invention that they are also advantageously characterized by the Heywood sphericity parameter multiplied by their size. This is because the Heywood sphericity parameter makes it possible to consider the shape of the said particles.

This parameter is calculated in the following way:

$$\text{Heywood sphericity parameter} = \frac{Perimeter}{2\sqrt{\pi.Area}}$$

Thus, according to the present invention, advantageously at least 95% of the said guanine particles exhibit a Heywood sphericity parameter multiplied by the size D50 of the particle of less than 65 $\mu$m, in particular of less than 60 $\mu$m and better still of less than 55 $\mu$m.

According to a specific embodiment of the invention, the said guanine particles exhibit a Heywood sphericity parameter multiplied by the size of the particle of less than 65 $\mu$m, in particular of less than 60 $\mu$m and better still of less than 55 $\mu$m.

It should be noted that the guanine crystals in their native form are characterized in that at least 95% of them have a Heywood sphericity parameter multiplied by the size of the particle of greater than 75 $\mu$m.

According to the present invention, the said guanine particles can be included in the composition according to the invention in a content ranging from 0.1% to 50% by weight, in particular from 0.5% to 40% by weight and preferably from 1% to 30% by weight, with respect to the total weight of the composition.

According to a first embodiment, for a liquid composition, the said guanine particles can be included in the composition according to the invention in a content ranging from 0.1% to 30% by weight, in particular from 0.5% to 27% by weight and preferably from 1% to 25% by weight, with respect to the total weight of the composition.

According to a second embodiment, for a pulverulent composition, the said guanine particles can be included in the composition according to the invention in a content ranging from 20% to 50% by weight, in particular from 25% to 50% by weight and preferably from 30% to 50% by weight, with respect to the total weight of the composition.

Of course, a composition in accordance with the present invention can comprise, in addition to the non-interferential particles of guanine as are defined above, guanine crystals for their interference properties.

As indicated above, the guanine crystals under consideration according to the invention prove to be particularly advantageous for their high covering power. Without, for all that, expecting to achieve that of TiO$_2$, the covering power displayed by the guanine particles in accordance with the present invention proves to be significantly greater than that of calcium carbonate.

This covering power can in particular be characterized by the contrast ratio.

The contrast ratio is defined in particular in International Application WO 98/52534.

In order to calculate the contrast ratio, the procedure is carried out in the following way:

A mixture formed of 15% by weight, with respect to the total weight, of the colouring agent to be studied and, for the remainder, of the reference emulsion below is applied to a black opaque support and to a white opaque support, according to a film with a thickness of 50 $\mu$m. This film is dried at 25°C +/- 1°C for 24 hours under a pressure of 1 atm.

The reference emulsion has the formulation:

|  | % by weight |
|---|---|
| - Water | 45.83 |
| - Methyl p-hydroxybenzoate | 0.45 |
| - Chlorphenesin | 0.34 |
| - Disodium EDTA | 0.11 |
| - Glycerol | 5.62 |
| - PEG-8 | 2.25 |
| - PEG-20 | 1.12 |
| - Magnesium aluminium silicate | 0.9 |
| - Sodium lauroyl sarcosinate | 1.68 |
| - Titanium dioxide (and) alumina (and) glycerin (and) silica | 3.37 |
| - Triethanolamine | 1.35 |
| - Stearic acid | 2.7 |
| - Glyceryl stearate | 2.02 |
| - Butyl p-hydroxybenzoate | 0.17 |
| - Isononyl isononanoate | 8.99 |
| - Cyclohexasiloxane | 6.57 |
| - Dimethicone | 10.28 |
| - Bis-PEG-15 methyl ether dimethicone | 2.25 |
| - Talc | 1.12 |
| - Kaolin | 1.12 |
| - Polymethyl methacrylate | 1.69 |

Using a colourimeter, for example of the Minolta CR-200 type, in illuminant D65 mode, angle of view 0°, the value of the tristimulus Y of the composition is measured at three different points of the black support and at three different points of the white support.

The contrast ratio corresponds to the mean of the three values of Y measured on the black support, divided by the mean of the three values of Y measured on the white support, and multiplied by 100.

The greater the contrast ratio and the closer to 100%, the greater the opaqueness of the colouring agent. The smaller the contrast ratio, the greater the transparency of the colouring agent.

As illustrated in the examples below, guanine particles in accordance with the invention can have a contrast ratio of approximately 45%, whereas natural guanine and synthetic guanine respectively have a contrast ratio of the order of 30% and 25%.

## Process for the preparation of the particles

As specified above, the guanine particles in accordance with the invention exhibit a specific size and a specific shape.

They are obtained from natural guanine particles which are milled.

According to the invention, the natural guanine particles are subjected to at least one dry milling stage.

Use may be made, among the various dry milling processes, of frictional or collision powder mills or a combination of these, making it possible to obtain fine to very fine milling grades (D50 < 30 $\mu$m, indeed even < 15 $\mu$m), such as air (or neutral gas) jet mill or pin mill, in particular the air jet mill.

According to the invention, use is made of air jet or pin mills, more preferably of the air jet mill.

The principal of the powder mills of air jet type is as follows: the material(s) to be milled is or are entrained into a milling chamber by the feed compressed air. Streams of compressed air or of another neutral gas, entering the milling chamber in a contrary direction via nozzles (micronization compressed air), accelerate the particles, which collide with one another in a region of maximum turbulence. The powders obtained, which can reach a size of the order of a few tens of to a few microns, are subsequently recovered by cyclone systems.

Mention may in particular be made, as commercial references for mills for dry milling, of the Chrispro Jet Mill air jet mill, manufactured by Micro-Macinazione S.A. (Italy), and the Hosokawa pin mill, supplied by Alpine A.G. (Germany).

Use will preferably be made of an air jet mill.

A person skilled in the art will use the powder mills according to the supplier's recommendations.

Thus, for the air jet mill:

- the feed pressure will be adjusted in order to ensure a supply of powder at a uniform flow rate;
- the value of the micronization pressure, which makes it possible to feed with air (or neutral gas) at very high speed via the peripheral nozzles, will be adjusted as a function of the target particle size measurement; and
- the flow rate will depend on the type of mixing and on the capacity of the mill.

For example, for the Jet Mill air jet mill, use is generally made of a feed pressure of 4 to 8 bar, in particular of 5 to 7 bar, a micronization pressure of 3 to 6 bar, in particular of 3.5 to 5.5 bar, and a flow rate which will depend on the type of mixing and on the capacity of the mill.

The process for the preparation of the milled natural guanine according to the invention consists of the following stages:

- a stage of milling the natural guanine particles in a powder mill by air jet mill or pin mill; and
- a stage of drying, in particular by heating, preferably via placing in an oven at a temperature of greater than or equal to 60°C for a period of time ranging from 1 to 8 hours and/or by bringing into contact with a stream of dry air.

The milling stage makes it possible to delaminate the structure and thus to detrimentally change the pearlescence effect inherent in the natural guanine. The milling can in particular be carried out using an air jet mill of Chrispro Jet Mill type from Micro-Macinazione S.A. and Micro-Grinding Ltd.

During the milling of the guanine, it should be noted that there is advantageously no caking effect, that is to say that there is no phenomenon of agglomeration of the guanine particles with one another.

The drying stage makes it possible remove the water and to thus increase the covering power of the guanine. This stage can in particular be carried out using an oven.

The conventional "Alpine" and "Air" milling processes advantageously make it possible to obtain guanine particles in accordance with the invention. One of them is described in detail below in Example 1.

## Additional natural compounds

In addition to the milled guanine above, the composition according to the invention can comprise at least one other additional natural compound or compound of natural origin.

## Physiologically acceptable medium

The composition under consideration according to the invention must be cosmetically or dermatologically acceptable, namely must comprise a non-toxic physiologically acceptable medium which is capable of being applied to human keratinous substances, in particular the skin, nails or lips. Within the meaning of the invention, the term "cosmetically acceptable" is understood to mean a composition having a pleasant appearance, pleasant odour and pleasant feel.

The composition employed according to the invention can be provided in any formulation form normally used in the cosmetics field and it can in particular be in the form of a suspension, dispersion, gel, serum, emulsion (W/O, O/W, multiple emulsion) which is fluid or solid, aqueous, anhydrous or greasy stick, solid, liquid or pasty anhydrous product, loose or compact powder, or cast, moulded or extruded form.

According to a specific form, the composition is an emulsion.

According to a specific form, the composition is a gel.

According to a specific form, the composition is a solid anhydrous product.

According to a specific form, the composition is a liquid or pasty anhydrous product.

According to a specific form, the composition is a powder.

According to a specific form, the composition is a cast product.

The physiologically acceptable medium can also in particular comprise organic solvents, optionally water and/or oils, in particular for compositions in the fluid or pasty form.

The organic solvent can be chosen, for example, from lower alcohols comprising from 1 to 4 carbon atoms, such as ethanol, isopropanol, propanol or butanol; polyethylene glycols having from 6 to 80 ethylene oxide units; polyols, such as propylene glycol, isoprene glycol, butylene glycol, glycerol, and their mixtures.

It can also comprise oils and optionally one or more other fatty substances.

Mention may be made, as oils which can be used in the invention, of mineral oils (liquid petrolatum), vegetable oils, animal oils (perhydrosqualene), synthetic oils, non-volatile or volatile silicone oils, and fluoro oils (perfluoropolyethers). Use may also be made, as fatty substances, of fatty alcohols, fatty acids or waxes.

According to a specific form, the composition comprises at least one organic solvent.

According to a specific form, the composition comprises at least one oil.

According to an alternative form of the invention, the composition employed according to the invention has less than 5% by weight, in particular less than 3% by weight and more particularly less than 1% by weight of water, indeed even is devoid of water, that is to say anhydrous.

According to another alternative form of the invention, the composition of the invention comprises an aqueous phase.

The aqueous phase (water and optionally the water-miscible solvent) can be present in a content ranging from 5% to 95% by weight, preferably from 10% to 85% by weight and better still from 2% to 80% by weight, with respect to the total weight of the composition.

The continuous aqueous phase can be composed essentially of water; it can also comprise a mixture of water and of water-miscible solvent (miscibility in water of greater than 50% by weight at 25°C), such as lower monoalcohols having from 1 to 5 carbon atoms, such as ethanol or isopropanol, glycols having from 2 to 8 carbon atoms, such as propylene glycol, ethylene glycol, 1,3-butylene glycol or dipropylene glycol, $C_3$-$C_4$ ketones, $C_2$-$C_4$ aldehydes and their mixtures.

Generally, the composition according to the invention comprises at least one non-aqueous solvent phase.

This phase is capable of forming a continuous phase and comprises, as its name indicates, at least one non-aqueous organic solvent which can be volatile or non-volatile.

The term "non-volatile non-aqueous organic solvent" is understood to mean a non-aqueous organic solvent which remains on the skin or the keratinous fibre at ambient temperature and atmospheric pressure. More specifically, a non-volatile non-aqueous organic solvent exhibits an evaporation rate of strictly less than 0.01 mg/cm$^2$/min.

In order to measure this evaporation rate, 15 g of the non-aqueous organic solvent to be tested are introduced into a crystallizing dish with a diameter of 7 cm placed on a balance in a large chamber of approximately 0.3 m$^3$ which is regulated in temperature, at a temperature of 25°C, and regulated in hygrometry, at a relative humidity of 50%. The liquid is allowed to freely evaporate, without stirring it, while providing ventilation by means of a fan (Papst-Motoren, reference 8550 N, rotating at 2700 revolutions per minute) placed in a vertical position above the crystallizing dish containing the said non-aqueous organic solvent, the blades being directed towards the crystallizing dish and at a distance of 20 cm with respect to the bottom of the crystallizing dish. The weight of non-aqueous organic solvent remaining in the crystallizing dish is measured at regular intervals. The evaporation rates are expressed in mg of non-aqueous organic solvent evaporated per unit of surface area (cm$^2$) and per unit of time (minute).

The term "volatile non-aqueous organic solvent" is understood to mean any non-aqueous medium which is capable of evaporating from the skin or the lips in less than one hour, at ambient temperature and atmospheric pressure. More specifically, a non-aqueous organic solvent exhibits an evaporation rate of between 0.01 and 200 mg/cm$^2$/min, limits included.

Thus, a composition according to the invention can comprise a liquid fatty phase.

In particular, the fatty phase of the composition can comprise at least one volatile or non-volatile oil chosen in particular from:

- hydrocarbon oils of animal origin, such as perhydrosqualene;
- hydrocarbon oils of vegetable origin, such as liquid triglycerides of carbon fatty acids, such as olive oil, rapeseed oil and their modified versions, such as hydrogenated olive oil stearyl esters and the liquid fraction of shea butter;
- synthetic esters and ethers, in particular of fatty acids, such as oils of formulae $R^1COOR^2$ and $R^1OR^2$ in which $R^1$ represents the residue of a fatty acid comprising from 8 to 29 carbon atoms and $R^2$ represents a branched or unbranched hydrocarbon chain comprising from 3 to 30 carbon atoms, such as, for example, purcellin oil, isononyl isononanoate, isopropyl myristate, 2-ethylhexyl palmitate, 2-octyldodecyl stearate, 2-octyldodecyl erucate or isostearyl isostearate; hydroxylated esters, such as isostearyl lactate, octyl hydroxystearate, octyldodecyl hydroxystearate, diisostearyl malate, triisocetyl citrate, fatty alcohol heptanoates, octanoates and decanoates; polyol esters, such as propylene glycol dioctanoate, neopentyl glycol diheptanoate and diethylene glycol diisononanoate; and pentaerythritol esters, such as pentaerythrityl tetraisostearate; lauric/palmitic/cetylic/stearic acid glycerides, isopropyl isostearate, alpha-D-glucopyranoside, beta-D-fructofuranosyl, acetate 2-methylpropanoate;
- linear or branched hydrocarbons, of mineral or synthetic origin, such as volatile or non-volatile liquid paraffins, and their derivatives, petrolatum, polydecenes or hydrogenated polyisobutene, such as parleam oil;
- fatty alcohols having from 8 to 26 carbon atoms, such as cetyl alcohol, stearyl alcohol and their mixture (cetearyl alcohol), octyldodecanol, 2-butyloctanol, 2-hexyldecanol, 2-undecylpentadecanol, oleyl alcohol, linoleyl alcohol or 2-octyldecanol;
- partially hydrocarbon-based and/or silicone-based fluoro oils, such as those described in the document JP-A-2-295 912;
- silicone oils, such as volatile or non-volatile polymethylsiloxanes (PDMSs) or phenyl(trimethylsiloxy)trisiloxane with a linear or cyclic silicone chain, which are liquid or pasty at ambient temperature, in particular cyclopolydimethylsiloxanes (cyclomethicones), such as cyclohexasiloxane; polydimethylsiloxanes comprising alkyl, alkoxy or phenyl groups, which are pendent or at the end of the silicone chain, which groups have from 2 to 24 carbon atoms; or phenylated silicones, such as phenyl trimethicones, phenyl dimethicones, phenyl(trimethylsiloxy)diphenylsiloxanes, diphenyl dimethicones, diphenyl(methyldiphenyl)trisiloxanes, (2-phenylethyl)trimethylsiloxysilicates and polymethylphenylsiloxanes; and

- their mixtures.

Mention may furthermore be made, by way of illustration of the other non-aqueous organic solvents which can also be employed according to the invention, of alcohols, polyols, polyol ethers and mixtures of these.

The alcohols can be chosen from lower $C_1$-$C_6$ alkanols and preferably chosen from ethanol, propanol and isopropanol.

The polyols can be chosen from glycerol, propylene glycol, polyethylene glycol, hexylene glycol, glycerol and pentanediol.

These solvents are more particularly advantageous in the formulation of compositions dedicated to an application on the nails.

It is understood that the compositions according to the invention can also comprise additional cosmetic ingredients conventionally employed in the formulation of specific formulation forms generally adjusted from the viewpoint of the keratinous substance targeted. This or these additional cosmetic ingredients can be chosen in particular from waxes, pasty fatty substances, film-forming polymers, non-ionic, anionic or cationic surfactants, hydrophilic or lipophilic gelling agents, hydrophilic or lipophilic active agents, preservatives, antioxidants, solvents, fragrances, fillers, sunscreens, bactericides, odour absorbers, additional colourants (for example: pigments, pearlescent agents, water-soluble or fat-soluble dyes), salts and their mixtures.

Thus, a cosmetic composition of the invention can comprise at least non-interferential particles of guanine as defined above and at least one additional cosmetic ingredient chosen from waxes, pasty fatty substances, film-forming polymers, non-ionic, anionic or cationic surfactants, hydrophilic or lipophilic gelling agents, hydrophilic or lipophilic cosmetic active agents, preservatives, antioxidants, solvents, fragrances, fillers, sunscreens, bactericides, odour absorbers, additional colourants (for example: pigments, pearlescent agents, water-soluble or fat-soluble dyes), salts and their mixtures.

The amounts of the additional cosmetic ingredients are those conventionally used in the field under consideration, for example from 0.01% to 20% of the total weight of the composition and preferably from 0.01% to 10% of the total weight of the composition.

According to a specific form, the composition of the invention comprises at least one wax.

According to a specific form, the composition of the invention comprises at least one pasty fatty substance.

According to a specific form, the composition of the invention comprises at least one film-forming polymer.

According to a specific form, the composition of the invention comprises at least one gelling agent, in particular for the fatty phase.

According to a specific form, the composition of the invention comprises at least one surfactant.

According to a specific form, the composition of the invention comprises at least one additional colourant.

According to a specific form, the composition of the invention comprises at least one filler.

Of course, a person skilled in the art will take care to choose the optional additional ingredients and/or their amounts so that the advantageous properties of the composition according to the invention are not, or not substantially, detrimentally affected by the envisioned addition.

*Additional colourants*

As specified above, the compositions according to the invention can comprise at least one additional colourant.

These colourants can advantageously be chosen from colourants which are "natural" or "of natural origin".

However, it is, of course, possible to combine synthetic colourants with these.

The composition according to the invention advantageously comprises less than 10% by weight of $TiO_2$, with respect to the total weight of white pigments having a colour index, in particular less than 8% by weight of $TiO_2$, with respect to the total weight of white pigments having a colour index, and more particularly less than 5% by weight of $TiO_2$, with respect to the total weight of white pigments having a colour index. More advantageously still, the composition according to the invention is devoid of $TiO_2$.

Mention may in particular be made, by way of illustration of colourants which can be present in the composition according to the invention, of the lipophilic dyes, hydrophilic dyes, pigments and pearlescent agents normally used in cosmetic or dermatological compositions, and their mixtures.

The fat-soluble dyes are, for example, Sudan Red, DC Red 17, DC Green 6, β-carotene, soybean oil, Sudan Brown, DC Yellow 11, DC Violet 2, DC Orange 5 and quinoline yellow.

The pigments can be white or coloured, inorganic and/or organic and coated or uncoated. Mention may be made, among the inorganic pigments, of zirconium oxide or cerium oxide, and also iron oxide or chromium oxide, manganese violet, ultramarine blue, chromium hydrate and ferric blue. Mention may be made, among the organic pigments, of carbon black, pigments of D&C type and lakes based on cochineal carmine of barium, strontium, calcium or aluminium.

The pearlescent pigments can be chosen from white pearlescent pigments, such as mica covered with titanium dioxide or with bismuth oxychloride, coloured pearlescent pigments, such as titanium dioxide-coated mica with iron oxides, titanium dioxide-coated mica with in particular ferric blue or chromium oxide, or titanium dioxide-coated mica with an organic pigment of the abovementioned type, and also pearlescent pigments based on bismuth oxychloride.

The pigments may have been subjected to a surface treatment.

Mention may also be made, among the pigments which can be used in the composition, of goniochromatic pigments.

Throughout the description, including the claims, the expression "comprising a" should be understood as being synonymous with "comprising at least one", unless otherwise specified.

The expressions "between ... and ..." and "ranging from ... to ..." should be understood as meaning limits included, unless otherwise specified.

The examples and figures which follow are presented by way of illustration and without implied limitation of the invention.

The present text also describes non-interferential particles of guanine having a size of less than 30 $\mu$m, preferably of less than 15 $\mu$m, with advantageously at least 95% of the said guanine particles exhibiting a Heywood sphericity parameter multiplied by the size of the particle of less than 65 $\mu$m, in particular of less than 60 $\mu$m and better still of less than 55 $\mu$m.

It also describes to a process for the preparation of guanine particles as defined above, comprising in particular the following stages:

- a stage of milling the natural guanine particles in a powder mill, in particular an air jet mill; and
- a stage of drying, in particular by heating, preferably via placing in an oven at a temperature of greater than or equal to 60°C for a period of time ranging from 1 to 8 hours and/or by bringing into contact with a stream of dry air.

## Description of the figures

The invention may be better understood on reading the detailed description of non-limiting implementational examples thereof and on examining the appended drawing, in which:

Figure 1 represents a photograph of guanine particles in accordance with the invention obtained after a stage of delaminating with milling devices.

Figure 2a represents the particle size distribution of the guanine particles before the dry-route delaminating process.

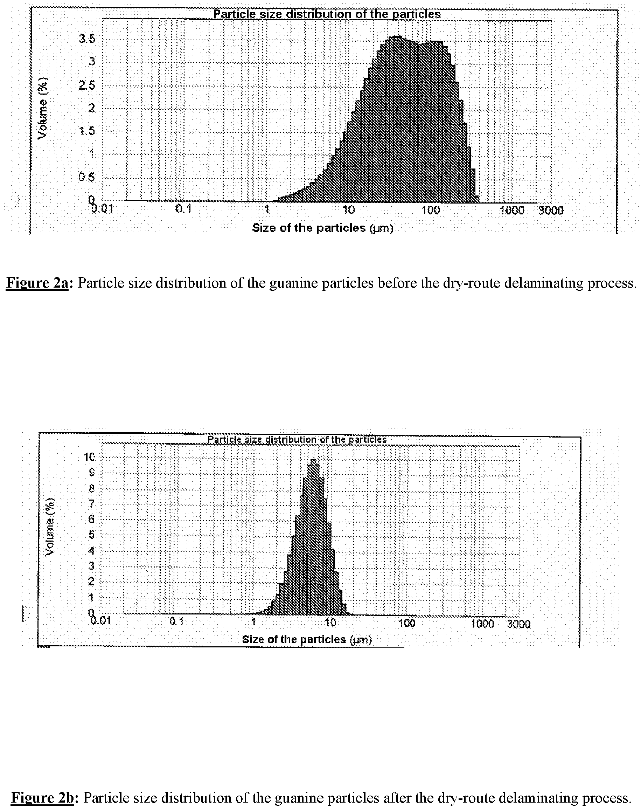

The characteristics of Figure 2a are:
Concentration: 0.0166 Vol%; volume-average D[4,3]: 77.156 $\mu$m; specific surface: 0.247 m$^2$/g; span (10% - 90%): 3.637; surface-average D[3,2]: 24.245 $\mu$m; uniformity: 1.11; type of distribution: Volume; d(0.1): 10.528 $\mu$m; d(0.5): 49.051 $\mu$m; d(0.9): 188.906 $\mu$m.

Figure 2b represents the particle size distribution of the guanine particles after the dry-route delaminating process.

The characteristics of Figure 2b are:
Concentration: 0.0032 Vol%; volume-average D[4,3]: 6.137 $\mu$m; specific surface: 1.21 m$^2$/g; span (10% - 90%): 1.200; surface-average D[3,2]: 4.976 $\mu$m; uniformity: 0.373; type of distribution: Volume; d(0.1): 3.021 $\mu$m; d(0.5): 5.702 $\mu$m; d(0.9): 9.864 $\mu$m.

## Examples

### Example 1: preparation of the guanine particles in accordance with the invention

Use of an air jet mill of Chrispro Jet Mill type from either of Micro-Macinazione S.A. and Micro-Grinding Ltd.

## Procedure

Adjustment of the metering device for dispensing the powder:

Flow rate: 2.1 kg/h
Charge: 363 g
Hopper retention: +/- 2 g
Sieve mesh size: 250 $\mu$m

Adjustment of the mill:

Compressed air feed pressure: 6 bar
Micronization pressure: 5 bar

The powder is introduced into the mill under these conditions. After the milling stage, the transformed powder is recovered.

The powder is subsequently subjected to a stage of drying by placing in an oven at 60°C for one hour.

As is shown in Figures 2a and 2b, this process makes it possible to considerably reduce the size of the guanine particles.

Thus, the natural guanine used, namely that distributed under the name Bioessence Pure Crystal by Bioingemar and exhibiting a D50 of 49 $\mu$m before delaminating, has, after milling, a D50 of 6 $\mu$m. Furthermore, the caking effect was not observed.

These specific features are confirmed in an identical fashion with an Alpine milling process.

**Example 2: measurement of the coverage (contrast ratio) for different pigments**

In order to confirm the coverage effects, the contrast ratios (CR) were measured according to the following protocol:

A mixture comprising 15% by weight of pigment in an emulsion (emulsion described in detail in the description) is milled on a triple roll mill and then spread at 50 $\mu$m over a contrast chart. 3 reflectance measurements are carried out using a Minolta CM-2002 spectrocolorimeter (D65/10°, specular component mode included). Only the tristimulus "Y" values are made use of for the calculation below:

$$CR = \frac{mean\ (Yblack)}{mean\ (Ywhite)} * 100$$

The greater the percentage of the contrast ratio, the greater the opaqueness of the sample.

The pigments tested and the results obtained are presented below.

| Pigment tested | Contrast ratio (%) |
|---|---|
| Mica | 6 |
| Barium sulfate | 28 |
| Calcium carbonate and starch | 9 |
| Talc | 9 |
| Magnesium stearate | 11 |
| Kaolinite | 13 |
| Synthetic guanine from Aceto France (D50 = 5.05 $\mu$m) | 23 |
| Bismuth oxychloride | 28 |
| Natural guanine Bioessence Pure Crystal from Bioingemar (D50 = 49 $\mu$m) | 29 |
| Calcium carbonate from Solvay (D50 = 4.98 $\mu$m) | 34 |
| Guanine of Example 1 (air guanine) (D50 = 6 $\mu$m) | 45 |
| Guanine obtained according to the "Alpine" milling process (alpine guanine) (D50 = 6 $\mu$m) | 44 |

First of all, this experiment clearly shows that the natural guanine is superior to the synthetic guanine since the corresponding contrast ratios are approximately 29% and 23% respectively (4% to 5% error).

This experiment also demonstrates the influence of the preparation of the guanine on the coverage. This is because the unmilled natural guanine exhibits a contrast ratio of approximately 29%, whereas the guanine according to Example 1 (air guanine) makes it possible to change this value to approximately 45%.

Moreover, as regards the relative positioning in comparison with calcium carbonate, it is found that the coverage of the guanine formulated according to the invention is greater by more than 10 units (i.e., an improvement of 30%). This is because the coverage value for calcium carbonate is 35%, which justifies its historical use since this value is the highest if the guanine according to the invention is not taken into account.

**Example 3: Composition of a pink lipstick**

| Component | Percentage by weight |
|---|---|
| Hydrogenated olive oil stearyl esters | 1.94 |
| Caprylic/capric acid triglycerides (60/40) | 2 |
| Lauric/palmitic/cetylic/stearic acid glycerides (50/20/10/10) | 2.68 |
| Isopropyl isostearate | 7 |
| Candelilla wax | 11 |
| Rice starch | 0.98 |
| alpha-D-Glucopyranoside, beta-D-fructofuranosyl, acetate 2-methylpropanoate | 29.72 |
| Hybrid rapeseed oil | 6.5 |
| Hydrogenated olive oil myristyl esters | 1.94 |
| Refined shea butter (*Butyrospermum parkii*) comprising approximately 7% of non-saponifiable products (M.p. 33°C) | 18.08 |
| Guanine particles prepared in Example 1 | 15.38 |
| Anthraquinone pigment alumina lake (Carmine CI 75470) | 0.77 |
| Sweet almond oil | 2.01 |

**Example 4: Composition of an orange lipstick**

| Component | Percentage by weight |
|---|---|
| Caprylic/capric acid triglycerides (60/40) | 7.83 |
| Calcium sulfate and calcium carbonate and iron hydroxide (natural earth) | 0.75 |
| Candelilla wax | 11 |
| Calcium sulfate and iron oxide (natural earth) | 2.25 |
| Stab. hybrid rapeseed oil (palmitic/stearic/oleic/linoleic acid triglycerides 3/6.5/81/6) (citric acid: 10 ppm) | 6.5 |
| Rice starch | 0.98 |
| Isopropyl isostearate | 7 |
| Hydrogenated olive oil stearyl esters (M.p. 57°) | 1.94 |
| Refined shea butter (*Butyrospermum parkii*) comprising approximately 7% of non-saponifiable products (M.p. 33°C) | 18.08 |
| Guanine particles prepared in Example 1 | 1.5 |

(continued)

| Component | Percentage by weight |
|---|---|
| Lauric/palmitic/cetylic/stearic acid glycerides (50/20/10/10) | 2.68 |
| Hydrogenated olive oil myristyl esters (M.p. 48°) | 1.94 |
| Sweet almond oil | 7.83 |
| alpha-D-Glucopyranoside, beta-D-fructofuranosyl, acetate 2-methylpropanoate | 29.72 |

## Example 5: Composition of a foundation

| Component | Percentage by weight |
|---|---|
| Tridecyl trimellitate | 11 |
| Liquid lanolin | 10 |
| Isostearyl malate | 13 |
| Acetylated lanolin | 11 |
| Lauric/palmitic/cetylic/stearic acid triglycerides (50/20/10/10) | 6 |
| Microcrystalline wax ($C_{20}$-$C_{60}$) | 4 |
| Protected isopropyl lanolate | 11 |
| 2-Octyldecanol | 16 |
| Phenyl(trimethylsiloxy)trisiloxane (viscosity: 20 cSt - MW: 372) | 5 |
| Polyethylene wax (MW: 500) | 8 |
| Guanine particles prepared in Example 1 | 5 |

## Example 6: Transparent gloss

| Component | Percentage by weight |
|---|---|
| 2-Octyldecanol | 12 |
| Di(tert-butyl)-4-hydroxytoluene | 0.07 |
| Polybutene (mono-olefins/isoparaffins 95/5) (MW: 2060) | 43 |
| Mixture of isopropyl, isobutyl and n-butyl p-hydroxybenzoates (40/30/30) | 0.6 |
| Pentaerythrityl tetraisostearate | 12.33 |
| Tridecyl trimellitate | 12 |
| 2-Decyltetradecanoic acid triglyceride (Guerbet $C_{24}$) | 17 |
| Guanine particles prepared in Example 1 | 3 |

## Example 7: Loose powder

| Component | Percentage by weight |
|---|---|
| Talc | 50.3 |

(continued)

| Component | Percentage by weight |
|---|---|
| Guanine particles prepared in Example 1 | 49.3 |
| Red iron oxide (CI: 77491) | 0.2 |
| Yellow iron oxide (CI: 77492) | 0.2 |

## Example 8: Compact powder

| Component | Percentage by weight |
|---|---|
| Talc | 23.1 |
| Mica | 13.3 |
| Maize starch | 17.3 |
| Guanine particles prepared in Example 1 | 36.41 |
| Yellow iron oxide (CI: 77492) | 0.74 |
| Red iron oxide (CI: 77491) | 0.6 |
| Black iron oxide (CI: 77499) | 0.25 |
| Octyldodecanol | 2 |
| *Argania spinosa* oil | 2 |
| *Simmondsia chinensis* oil | 2 |
| *Butyrospermum parkii* butter | 2 |
| Tocopherol | 0.1 |
| *Camellia sinensis* leaf extract | 0.2 |

## Example 9: Tinted cream (W/O emulsion)

| | |
|---|---|
| Sorbitan isostearate | 5 |
| Dicaprylyl carbonate | 28 |
| Propylparaben | 0.1 |
| Disteardimonium hectorite | 1 |
| Titanium oxide (CI: 77891) | 0.8 |
| Yellow iron oxide (CI: 77492) | 0.92 |
| Red iron oxide (CI: 77491) | 0.2 |
| Black iron oxide (CI: 77499) | 0.18 |
| Nylon 12 | 4.7 |
| Water | 45.7 |
| Glycerol | 3 |
| Sodium chloride | 0.7 |
| Preservatives | 0.7 |

(continued)

| | |
|---|---|
| Guanine particles prepared in Example 1 | 9 |

**Example 10: Nail varnish**

| Component | Percentage by weight |
|---|---|
| Nitrocellulose | 11 |
| N-Ethyl-o,p-toluenesulfonamide | 5 |
| Alkyd resin | 10 |
| Isopropanol | 4 |
| Guanine particles prepared in Example 1 | 5 |
| Butyl acetate/ethyl acetate 50/50 | q.s. for 100 |

**Claims**

1. Cosmetic composition for caring for and/or making up keratinous substances comprising, in a physiologically acceptable medium, non-interferential particles of guanine of natural origin having a D50 volume-average size ranging from 1 to 15 μm, said non-interferential particles of guanine of natural origin being prepared according to a process consisting of the following stages:

   - a stage of milling natural guanine particles in a powder mill by air jet mill or pin mill, and
   - a stage of drying.

2. Composition according to claim 1, **characterized in that** the said guanine particles originate from fish scales.

3. Composition according to any one of the preceding claims, **characterized in that** the said guanine particles are included in a content ranging from 0.1% to 50% by weight, in particular from 0.5% to 40% by weight and preferably from 1% to 30% by weight, with respect to the total weight of the composition.

4. Composition according to claim 1, **characterized in that** it has less than 5% by weight, in particular less than 3% by weight and more particularly less than 1% by weight of water, indeed even is devoid of water, that is to say anhydrous.

5. Composition according to any one of the preceding claims, **characterized in that** it comprises at least one additional colourant.

6. Composition according to any one of the preceding claims, **characterized in that** it comprises less than 10% by weight of $TiO_2$, with respect to the total weight of white pigments having a colour index, in particular less than 8% by weight of $TiO_2$, with respect to the total weight of white pigments having a colour index, and more particularly less than 5% by weight of $TiO_2$, with respect to the total weight of white pigments having a colour index.

7. Composition according to any one of the preceding claims, **characterized in that** it is a foundation.

8. Composition according to any one of the preceding claims, **characterized in that** it is a lipstick.

9. Composition according to claim 1, **characterized in that** it is in the form of a suspension, dispersion, gel, serum, emulsion (W/O, O/W, multiple emulsion) which is fluid or solid, aqueous, anhydrous or greasy stick, solid, liquid or pasty anhydrous product, loose or compact powder, or cast, moulded or extruded form.

10. Process for the preparation of non-interferential particles of guanine of natural origin having a D50 volume-average size ranging from 1 to 15 $\mu$m, consisting of the following stages:

- a stage of milling the natural guanine particles in a powder mill by air jet mill or pin mill; and
- a stage of drying.

11. Cosmetic method for caring for and/or making up keratinous substances, **characterized in that** a cosmetic composition according to any one of claims 1 to 9 is applied to the keratinous substances.

**Patentansprüche**

1. Kosmetische Zusammensetzung zum Pflegen und/oder Schminken von keratinösen Substanzen, umfassend in einem physiologisch verträglichen Medium nicht-interferierende Teilchen von Guanin natürlichen Ursprungs mit einer volumengemittelten D50-Größe in einem Bereich von 1 bis 15 $\mu$m, wobei die nicht-interferierenden Teilchen von Guanin natürlichen Ursprungs gemäß einem Verfahren hergestellt werden, das aus den folgenden Stufen besteht:

   - einer Stufe von Mahlen von natürlichen Guanin-Teilchen in einer Pulvermühle durch Luftstrahlmühle oder Stiftmühle, und
   - einer Stufe von Trocknen.

2. Zusammensetzung gemäß Anspruch 1, **dadurch gekennzeichnet, dass** die Guanin-Teilchen von Fischschuppen stammen.

3. Zusammensetzung gemäß einem der vorhergehenden Ansprüche, **dadurch gekennzeichnet, dass** die Guanin-Teilchen in einem Gehalt, der in einem Bereich von 0,1 Gew.-% bis 50 Gew.-%, insbesondere von 0,5 Gew.-% bis 40 Gew.-% und bevorzugt von 1 Gew.-% bis 30 Gew.-%, bezogen auf das Gesamtgewicht der Zusammensetzung, liegt, eingeschlossen sind.

4. Zusammensetzung gemäß Anspruch 1, **dadurch gekennzeichnet, dass** sie weniger als 5 Gew.-%, insbesondere weniger als 3 Gew.-% und stärker besonders weniger als 1 Gew.-% Wasser aufweist, tatsächlich sogar frei von Wasser, das heißt wasserfrei ist.

5. Zusammensetzung gemäß einem der vorhergehenden Ansprüche, **dadurch gekennzeichnet, dass** sie wenigstens ein zusätzliches farbgebendes Mittel umfasst.

6. Zusammensetzung gemäß einem der vorhergehenden Ansprüche, **dadurch gekennzeichnet, dass** sie weniger als 10 Gew.-% $TiO_2$, bezogen auf das Gesamtgewicht an Weißpigmenten mit einer Farbzahl, insbesondere weniger als 8 Gew.-% $TiO_2$, bezogen auf das Gesamtgewicht an Weißpigmenten mit einer Farbzahl, und stärker besonders weniger als 5 Gew.-% $TiO_2$, bezogen auf das Gesamtgewicht an Weißpigmenten mit einer Farbzahl, umfasst.

7. Zusammensetzung gemäß einem der vorhergehenden Ansprüche, **dadurch gekennzeichnet, dass** sie eine Grundierung ist.

8. Zusammensetzung gemäß einem der vorhergehenden Ansprüche, **dadurch gekennzeichnet, dass** sie ein Lippenstift ist.

9. Zusammensetzung gemäß Anspruch 1, **dadurch gekennzeichnet, dass** sie in der Form einer Suspension, einer Dispersion, eines Gels, eines Serums, einer Emulsion (W/O, O/W, multiplen Emulsion), welche fluid oder fest ist, eines wässrigen, wasserfreien oder fettigen Stifts, eines festen, flüssigen oder pastösen wasserfreien Produkts, eines losen oder kompakten Pulvers oder einer gegossenen, geformten oder extrudierten Form vorliegt.

10. Verfahren zur Herstellung von nicht-interferierenden Teilchen von Guanin natürlichen Ursprungs mit einer volumengemittelten D50-Größe in einem Bereich von 1 bis 15 $\mu$m, bestehend aus den folgenden Stufen:

    - einer Stufe von Mahlen der natürlichen Guanin-Teilchen in einer Pulvermühle durch Luftstrahlmühle oder Stiftmühle, und
    - einer Stufe von Trocknen.

**11.** Kosmetisches Verfahren zum Pflegen und/oder Schminken von keratinösen Substanzen, **dadurch gekennzeichnet, dass** eine kosmetische Zusammensetzung gemäß einem der Ansprüche 1 bis 9 auf die keratinösen Substanzen aufgebracht wird.

## Revendications

**1.** Composition cosmétique de soin et/ou de maquillage des matières kératiniques comprenant dans un milieu physiologiquement acceptable des particules non interférentielles de guanine naturelle ayant une taille moyenne en volume D50 située dans la plage allant de 1 à 15 $\mu$m, lesdites particules non interférentielles de guanine naturelle étant préparées conformément à un procédé consistant en les étapes suivantes :

- une étape de broyage des particules non interférentielles de guanine naturelle dans un broyeur à poudres par broyage à jet d'air ou broyage à pointes, et
- une étape de séchage.

**2.** Composition selon la revendication 1, **caractérisée en ce que** lesdites particules de guanine proviennent d'écaillés de poissons.

**3.** Composition selon l'une quelconque des revendications précédentes, **caractérisée en ce que** lesdites particules de guanine sont incluses en une proportion située dans la plage allant de 0,1 à 50 % en poids, en particulier de 0,5 à 40 % en poids et de préférence de 1 à 30 % en poids par rapport au poids total de la composition.

**4.** Composition selon la revendication 1, **caractérisée en ce qu'**elle a moins de 5 % en poids, en particulier moins de 3 % en poids, plus particulièrement moins de 1 % en poids d'eau, voire est exempte d'eau, c'est-à-dire anhydre.

**5.** Composition selon la revendication 1, **caractérisée en ce qu'**elle comprend au moins une matière colorante additionnelle.

**6.** Composition selon l'une quelconque des revendications précédentes, **caractérisée en ce qu'**elle comprend moins de 10 % en poids de $TiO_2$ par rapport au poids total de pigments blancs dotés d'un color index, en particulier moins de 8 % en poids de $TiO_2$ par rapport au poids total de pigments blancs dotés d'un color index, plus particulièrement moins de 5 % en poids de $TiO_2$ par rapport au poids total de pigments blancs dotés d'un color index.

**7.** Composition selon l'une quelconque des revendications précédentes, **caractérisée en ce qu'**il s'agit d'un fond de teint.

**8.** Composition selon l'une quelconque des revendications précédentes, **caractérisée en ce qu'**il s'agit d'un rouge à lèvres.

**9.** Composition selon l'une quelconque des revendications précédentes, **caractérisée en ce qu'**elle est sous forme de suspension, dispersion, gel, sérum, émulsion (E/H, H/E, émulsion multiple) qui est fluide ou solide, stick aqueux, anhydre ou gras, produit anhydre solide, liquide ou pâteux, poudre libre ou compacte, ou forme coulée, moulée ou extrudée.

**10.** Procédé pour la préparation de particules non interférentielles de guanine naturelle ayant une taille moyenne en volume D50 située dans la plage allant de 1 à 15 pm, consistant en les étapes suivantes :

- une étape de broyage des particules non interférentielles de guanine naturelle dans un broyeur à poudres par broyage à jet d'air ou broyage à pointes ; et
- une étape de séchage.

**11.** Procédé cosmétique de soin et/ou de maquillage des matières kératiniques, **caractérisé par le fait que** l'on applique sur les matières kératiniques, une composition cosmétique selon l'une quelconque des revendications 1 à 9.

**Figure 1**: Photograph of the guanine particles after the stage of delaminating by milling devices

**Figure 2a:** Particle size distribution of the guanine particles before the dry-route delaminating process.

**Figure 2b:** Particle size distribution of the guanine particles after the dry-route delaminating process.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 59067285 A
- US 3892844 A
- US 2005257718 A
- WO 9852534 A
- JP 2295912 A

**Non-patent literature cited in the description**

- Cosmetics and Toiletries Magazine. 2005, vol. 120, 10